# EUROPÄISCHE PATENTSCHRIFT

(11) **EP 0 585 751 B1**
(45) Veröffentlichungstag und Bekanntmachung des Hinweises auf die Patenterteilung: **23.10.1996**
(21) Anmeldenummer: 93113327.6
(22) Anmeldetag: 20.08.1993
(51) Int. Cl.: C07C 251/60, A01N 37/50, C07C 255/61, C07C 327/48, C07C 323/45

(54) **N-Methylamide, Verfahren und Zwischenprodukte zu ihrer Herstellung sowie Verfahren zur Bekämpfung von Schädlingen**
N-Methylamides, process and intermediates for their preparation and their use as pesticides
N-Méthylamides, procédé pour leur préparation et intermédiaires et leur utilisation comme pesticides

(30) Priorität: 29.08.1992 DE 4228867
(43) Veröffentlichungstag der Anmeldung: 09.03.1994
(73) Patentinhaber: BASF Aktiengesellschaft, 67063 Ludwigshafen (DE)
(72) Erfinder: Bayer, Herbert, Dr., D-6800 Mannheim 1 (DE); Wingert, Horst, Dr., D-6800 Mannheim 1 (DE); Sauter, Hubert, Dr., D-6800 Mannheim 1 (DE); Benoit, Remy, Dr., D-6700 Ludwigshafen (DE); Oberdorf, Klaus, Dr., D-6900 Heidelberg (DE); Roehl, Franz, Dr., D-6707 Schifferstadt (DE); Ammermann, Eberhard, Dr., D-6148 Heppenheim (DE); Lorenz, Gisela, Dr., D-6730 Neustadt (DE)

(56) Entgegenhaltungen:
- EP-A- 0 463 488
- WO-A-92/13830

## Beschreibung

Die vorliegende Erfindung betrifft neue N-Methylamide, Verfahren und Zwischenprodukte zu ihrer Herstellung sowie Verfahren zur Bekämpfung von Schädlingen, insbesondere von Pilzen, Insekten, Nematoden und Spinnmilben mit diesen Verbindungen.

Es ist bekannt, substituierte N-Methylamide als Schädlingsbekämpfungsmittel (vgl. EP-A 463 488, EP-A 398 692) zu verwenden. Ihre Wirkung ist jedoch unbefriedigend.

Es wurde überraschend gefunden, daß N-Methylamide der allgemeinen 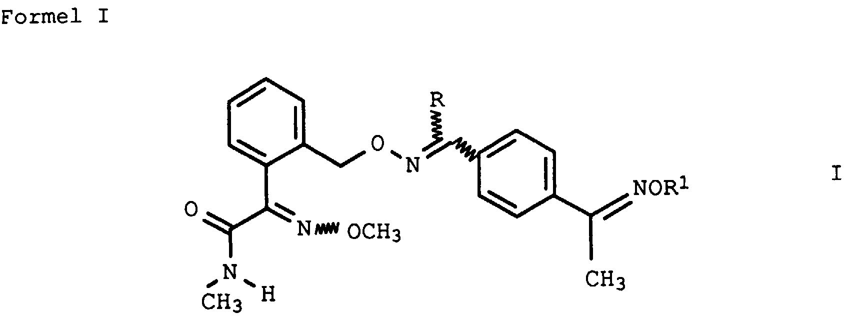 eine ausgezeichnete fungizide, insektizide, nematizide und akarizide Wirkung haben, die besser ist, als die der bekannten N-Methylamide.

Die fungizide Wirkung wird bevorzugt.

Die in der allgemeinen Formel I aufgeführten Reste können beispielsweise die folgende Bedeutung haben:
- R: Methyl;
- R¹: Methyl, Ethyl, Propyl, iso-Propyl, Butyl, iso-Butyl, sec.- Butyl und tert.-Butyl.

Die neuen Verbindungen der allgmeinen Formel I können aufgrund der C=N-Doppelbindungen als E/Z-Isomerengemische anfallen. Diese können in der üblichen Weise, z. B. durch Kristallisation oder Chromatographie, in die einzelnen Komponenten aufgetrennt werden. Sowohl die einzelnen isomeren Verbindungen als auch ihre Gemische werden von der Erfindung umfaßt und sind als Schädlingsbekämpfungsmittel brauchbar. In der Regel fallen die erfindungsgemäßen

Verbindungen jedoch weit überwiegend in der (E, E)-Konfiguration an. Diese Isomeren sind auch bezüglich ihrer Wirkung bevorzugt.

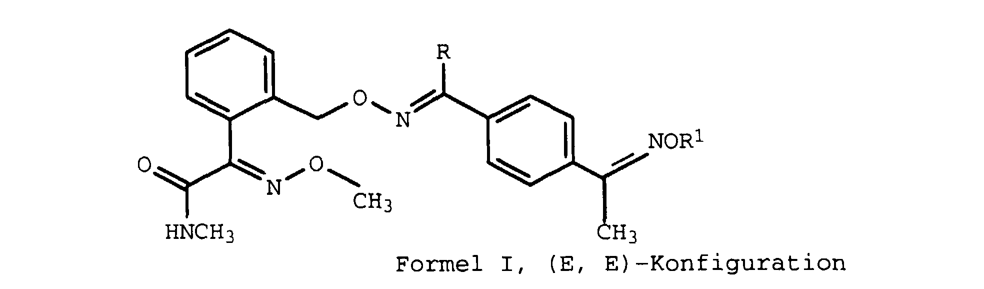

Die neuen Verbindungen der Formel I können beispielsweise wie folgt dargestellt werden:

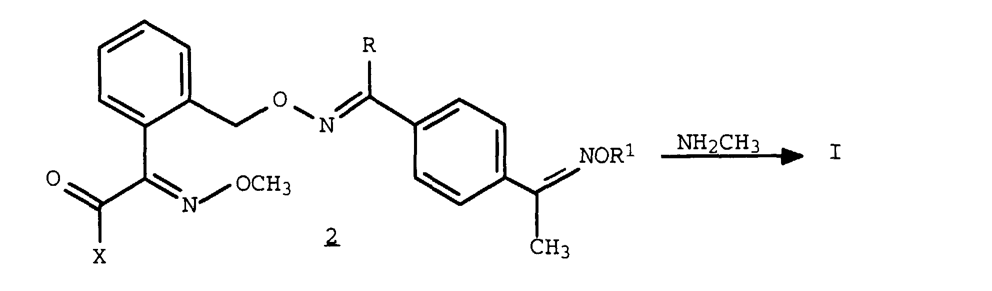

Ausgehend von den Verbindungen der Formel 2, mit X = OH, Cl und O(C₁-C₄-Alkyl) [bekannt aus EP-A 463 488, EP-A 472 300 und EP-A 426 460] lassen sich die erfindungsgemäßen Monomethylamide I analog zu literaturbekannten Methoden durch Umsetzung mit Methylamin erhalten (vgl. Organikum; 16. Auflage S. 408f (1985)).

Eine besonders vorteilhafte Möglichkeit zur Darstellung der Verbindungen I besteht darin, daß man das O-Benzylhydroxylamin 3 mit den Phenylketonen 4 umsetzt (vgl. D. Otzanak, J. Chem. Soc., Chem. Commun. 903, 1986). Das hierfür benötigte Zwischenprodukt 3, läßt sich überraschenderweise besonders glatt und vorteilhaft in einer einfachen Reaktion durch dreifache Aminolyse von 3a (mit Methylamin) herstellen.

Diese einstufige Reaktionsführung 3a → 3 vermeidet die übliche umständlichere und aufwendigere, zweistufige Vorgehensweise, z.B. mit Hilfe einer Hydrazinolyse die O-Benzylhydroxylamingruppe von der Phthalimidschutzgruppe freizusetzen und vorher oder anschließend die Methylestergruppe zum Methylamid umzuwandeln. Überraschend sind die hohen Ausbeuten dieser Reaktion, obwohl normalerweise recht unterschiedliche Bedingungen einerseits für die Aminolyse eines Methylesters zum Methylamid und andererseits für die Freisetzung eines O-Benzylhydroxylamins aus dem entsprechenden O-Benzylhydroxyphthalimid optimal sind.

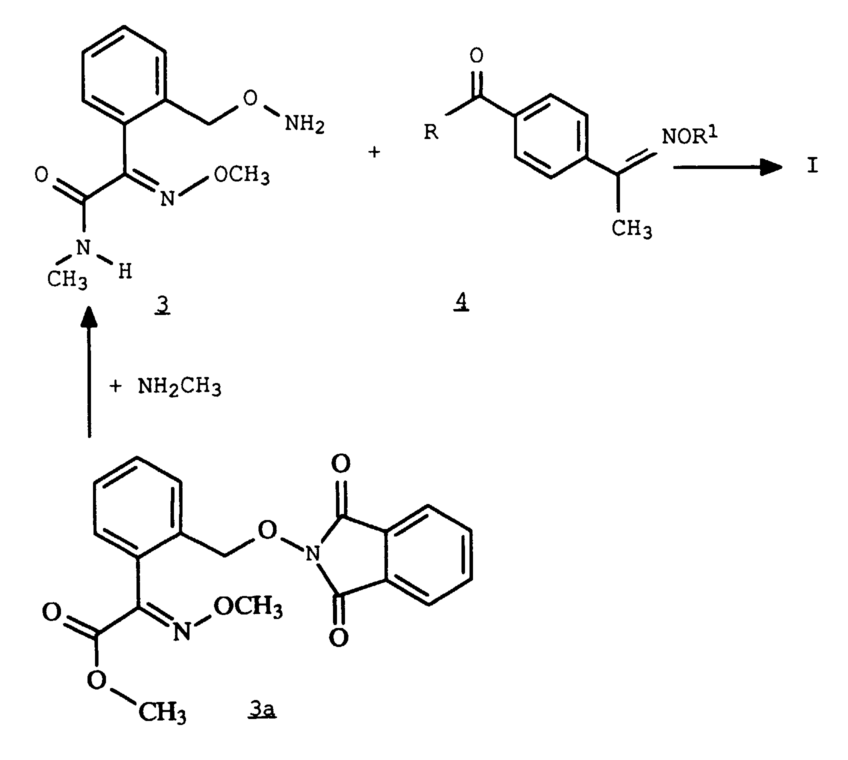

Die Phenylketone 9 sind bekannt oder können analog zu bekannten Verfahren hergestellt werden (Houben-Weyl, Bd 7/2a und 7/3a).

Man erhält die neuen Verbindungen der Formel I auch, indem man eine Monomethylamid-Benzylverbindung 5, in der L für eine Abgangsgruppe (z. B. Brom, Chlor, Jod, Mesylat, Tosylat, Triflat) steht (vgl. z.B. EP-A 398 692, EP-A 463 488) mit einem Oxim der Formel 6 umsetzt (vgl. z.B. Houbon-Weyl, Bd 10/1, S. 1186 f).

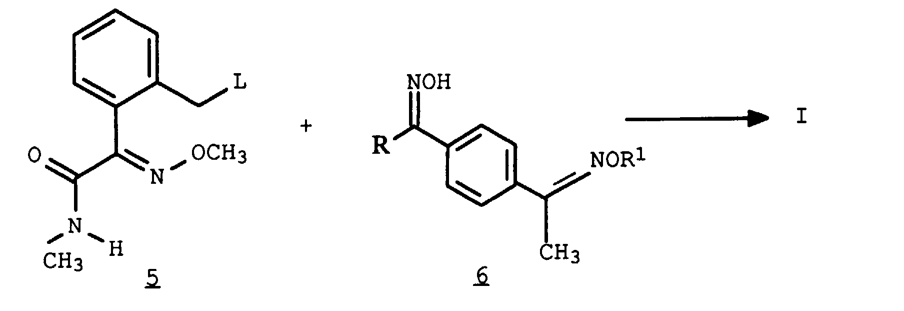

Die Oxime 6 sind bekannt oder können nach bekannten Verfahren hergestellt werden (vgl. Houben-Weyl, Bd 10/4; J. Med. Chem. 26, 1360 (1983)).

Weiterhin kann man die neuen Verbindungen der Formel I darstellen, indem man den Benzylalkohol 7 in an sich bekannter Weise mit den Oximen 6 umsetzt (vgl. J. Jurczak, Synthesis, 682, 1976).

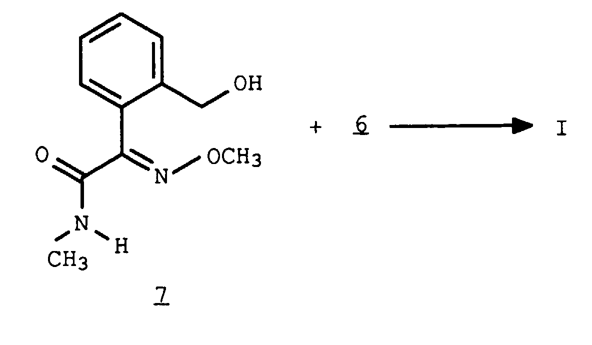

Alternativ hierzu lassen sich die Ketoamide 8 in an sich bekannter Weise mit Methoxyamin(-hydrochlorid) zu den neuen Verbindungen I umsetzen (vgl. z. B. DE-A 36 23 921).

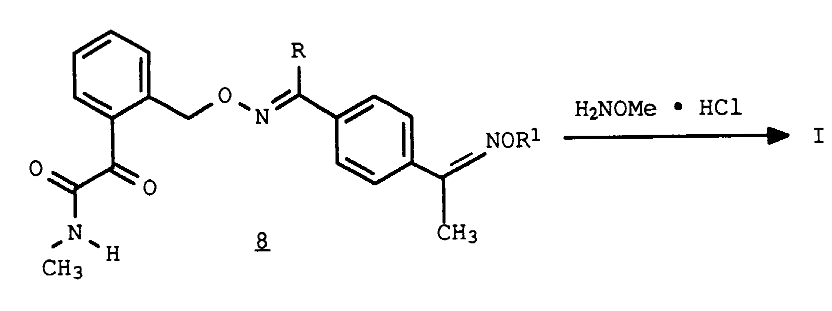

Die Ketoamide 8 selbst lassen sich beispielsweise aus den Ketoestern 9 durch Aminolyse erhalten, wobei die Ketoester 9 selbst entweder durch Pinnerreaktion aus den Benzoylcyaniden 10 oder durch Umsetzung der Oxime 6 mit den Benzylverbindungen 11 zugänglich sind.

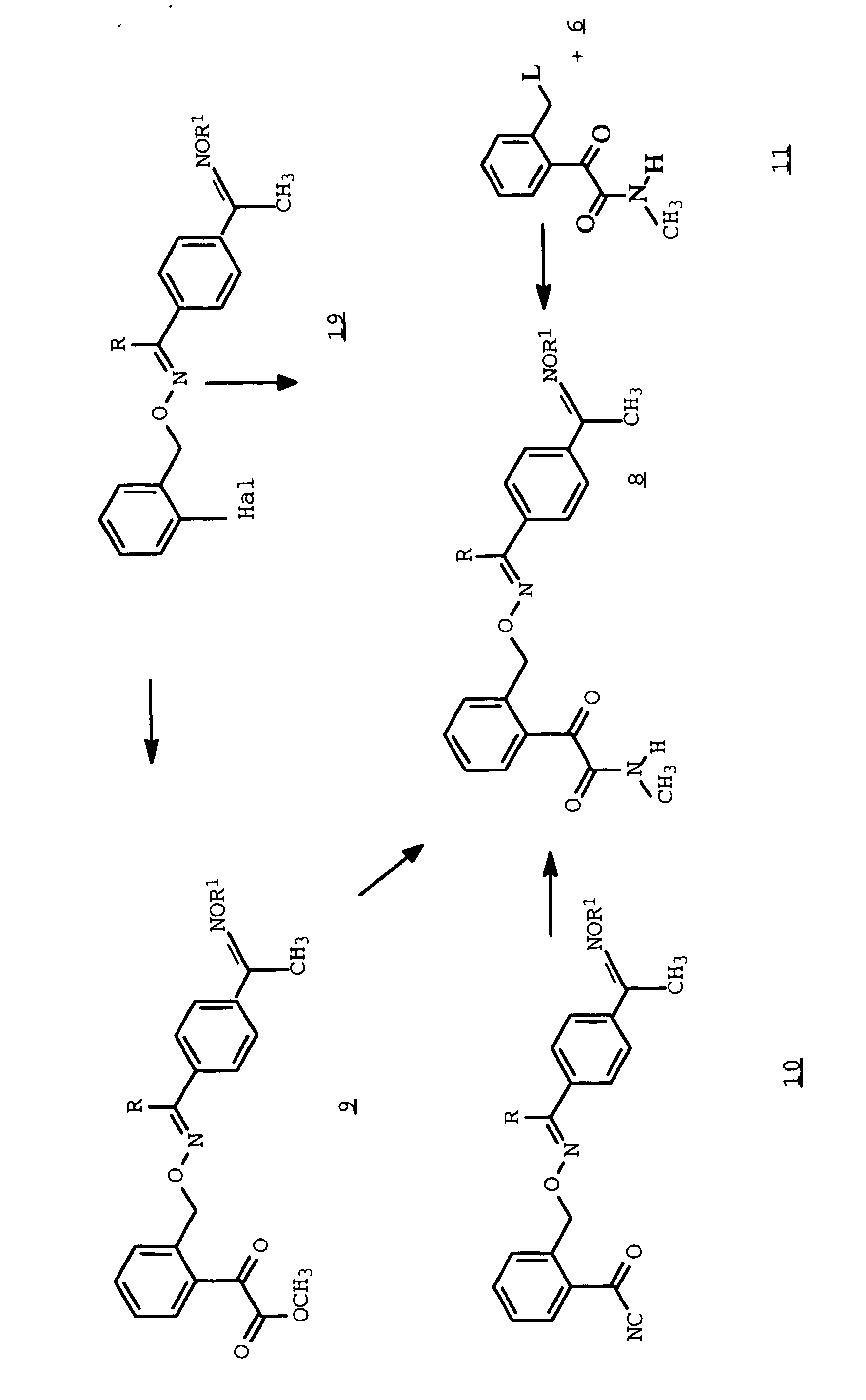

Als weitere Vorstufe zur Darstellung der Ketoamide 8 bzw. der Ketoester 9 sind die Arylhalogenide 19 (Hal = Chlor, Brom, Jod) geeignet. Diese lassen sich durch Metallierung (z.B. mit Magnesium, Methylmagnesiumbromid, Natrium, n-Buthyl-Lithium) in das entsprechende Metallorganyl überführen, das dann in an sich bekannter Weise mit Oxalsäurederivaten (wie z.B. Oxalsäurediester, Oxalsäureester-N-Methylamid) zu den Produkten 8 bzw. 9 umgesetzt werden kann (vgl. z.B. L. M. Weinstock, Synth. Commun. 11, 943 (1981)).

Auch die Cyanoximether 12 lassen sich in an sich bekannter Weise mit Methylamin in die neuen Verbindungen der Formel I umwandeln (vgl. EP-A 468 775).

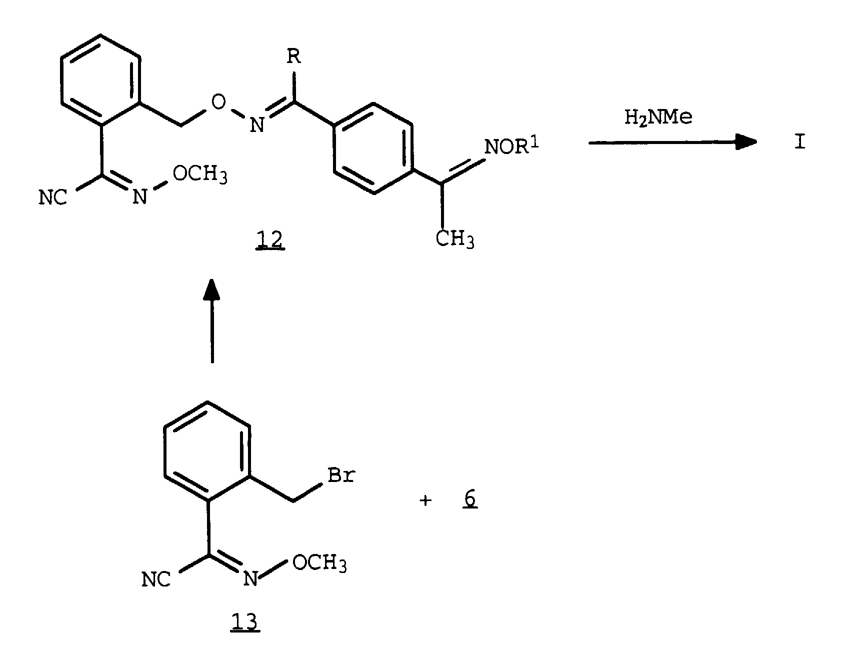

Die Cyanoximether 12 erhält man beispielsweise nach bekannten Verfahren durch Umsetzung der Oxime 6 mit dem Benzylbromid 13.

Die Ketoamide der Formel 8 lassen sich außerdem in einfacher Weise auch aus den Nitrilium-Salzen 17 durch Hydrolyse sowohl in saurer als auch in alkalischer Lösung gewinnen (vgl. Houben-Weyl, BdE5/Teil 2, S. 1580 f).

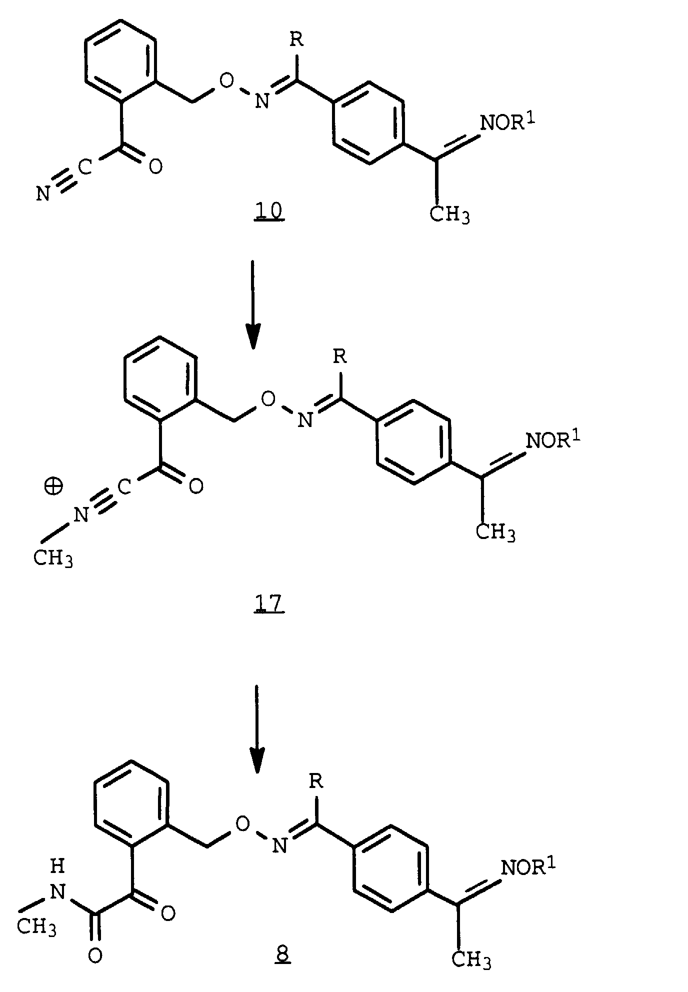

Die Nitrilium-Salze der Formel 17 sind beispielsweise leicht zugänglich aus den Verbindungen der Formel 10 durch Umsetzung mit Trimethyloxonium-Salzen wie z. B. Trimethyloxonium-tetrafluoroborat oder durch Umsetzung von 10 mit z. B. Trifluormethansulfonsäuremethylester (vgl. Houben-Weyl, Bd E5/Teil 2, S. 1573-6).

In entsprechender Weise lassen sich auch die Verbindungen der allgemeinen Formel I leicht aus Nitrilium-Salzen der Formel 18 durch Hydrolyse in saurer oder alkalischer Lösung gewinnen (vgl. Houben-Weyl, Bd E5/Teil 2, S. 1580 f).

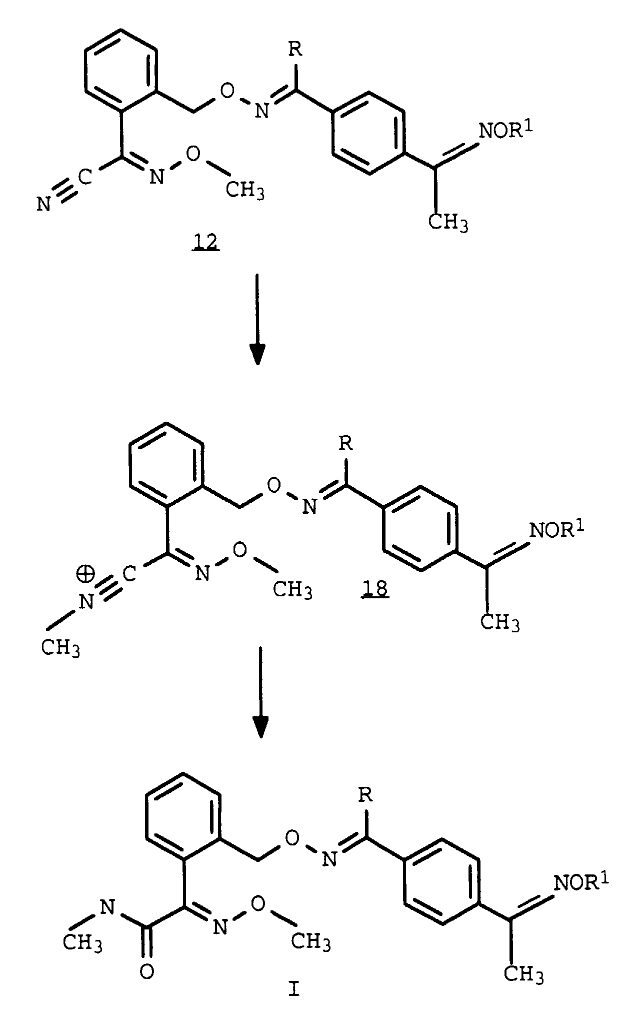

Die Nitrilium-Salze der Formel 18 wiederum sind zum Beispiel leicht zugänglich aus den Verbindungen der Formel 12 durch Umsetzung mit Trimethyloxonium-Salzen wie zum Beispiel Trimethyloxonium-tetrafluoroborat oder durch Umsetzung von 12 mit z. B. Trifluormethansulfonsäuremethylester (vgl. Houben-Weyl, Bd E5/Teil 2, S. 1573-6).

Ferner lassen sich die neuen Verbindungen der Formel I auch durch Methylierung geeigneter Zwischenstufen wie z. B. 14 - 16 darstellen.

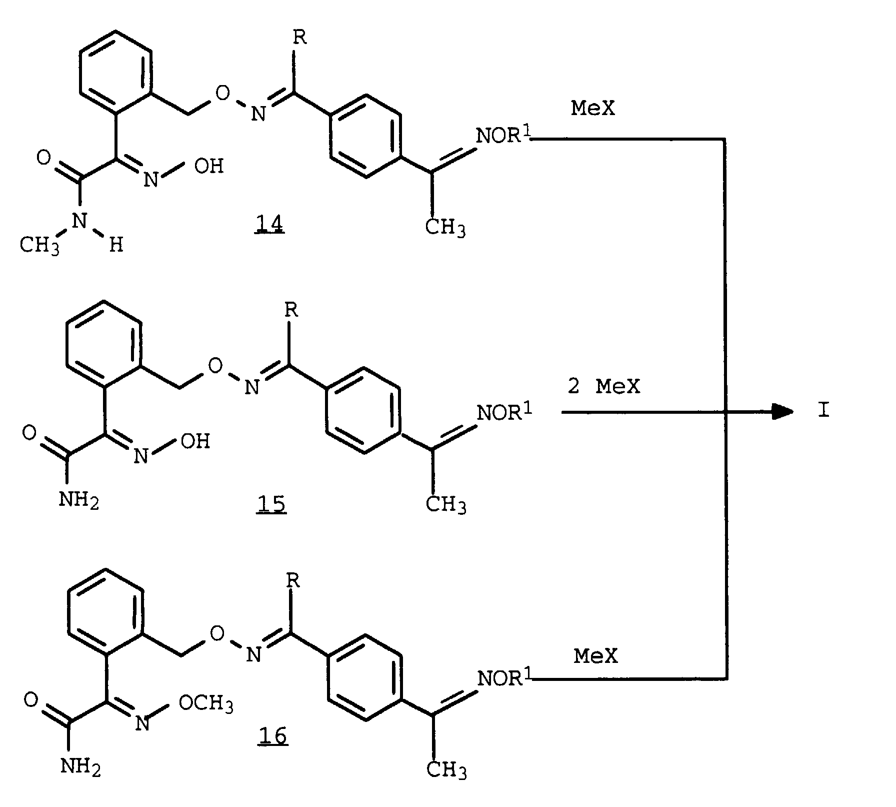

Die Alkylierung erfolgt zweckmäßigerweise in Gegenwart einer Base, wie z. B. KOH, K₂CO₃ oder Triethylamin. Als Alkylierungsmittel lassen sich Methylhalogenide oder auch Dimethylsulfat verwenden (vgl. z. B. Houben-Weyl, Bd X/1 S. 1186 f; bzw. G. L. Isele, Synthesis, 266 (1971)).

Die in den Formeln 2 - 19 aufgeführten Substituenten R und Am haben die in Anspruch 1 angegebene Bedeutung. Der Substituent L bezeichnet eine Abgangsgruppe wie z. B. Halogen (Chlor, Brom, Jod), Tosylat, Mesylat oder Triflat.

Die folgenden Beispiele sollen die Herstellung der neuen Wirkstoffe und des neuen Zwischenproduktes erläutern:

### Beispiel 1

### Darstellung von E-2-Methoxyimino-2-[(2'-phthalimidooxymethyl)phenyl]essigsäuremethylester

Zu einer Lösung von 150 g (0,52 mol) 2-Methoxyimino-2-[(2'-brommethyl)phenyl]essigsäuremethylester und 85 g (0,52 mol) N-Hydroxyphthalimid in 350 ml N-Methylpyrrolidon tropft man 59 g (0,58 mol) Triethylamin und rührt 2 h bei 70°C. Man gießt das Reaktionsgemisch auf 2 1 Eiswasser, saugt die ausgefallenen Kristalle ab und nimmt sie in Methylenchlorid auf. Nach Waschen der organischen Phase mit Wasser, Trocknen über Natriumsulfat und Abrotieren des Lösungsmittels erhält man 168 g (88 % Ausbeute) E-2-Methoxyimino-2-[(2'-phthalimidooxymethyl)phenyl]essigsäuremethylester als hellgraues Pulver vom Schmelzpunkt 152 - 153°C.
- ¹H-NMR (CDCl₃): δ =: 3,81 (s, 3H); 3,95 (s, 3H); 5,05 (s, 2H); 7,12 - 7,81 (m, 8H) ppm.

### Beispiel 2

### Darstellung von E-2-Methoxyimino-2-[(2'-aminooxymethyl)phenyl]essigsäuremonomethylamid

168 g (0,46 mol) E-2-Methoxyimino-2-[(2'-phthalimidooxymethyl)phenyl]essigsäuremethylester werden mit 1 1 40 %iger wäßriger Monomethylaminlösung versetzt und 4 h bei 40°C gerührt. Nach Abkühlen wird mit Methylenchlorid extrahiert, die organische Phase mit Wasser gewaschen, über Natriumsulfat getrocknet und eingeengt. Nach Kristallisation des Rückstandes aus Methyl-tert.- Butyl-Ether/n-Hexan erhält man 92 g (84 % Ausbeute) E-2-Methoxyimino-2- [(2'-aminooxymethyl)phenyl]essigsäuremonomethylamid als hellgelbe Kristalle vom Schmelzpunkt 78 - 81°C. Die Mutterlauge enthält weiteres Endprodukt.
- ¹H-NMR (CDCl₃): δ =: 2,91 (d, 3H); 3,94 (s, 3H); 4,58 (s, 2H); 5,28 (s, br, 2H); 6,88 (s, br, 1H); 7,14 - 7,43 (m, 4H) ppm.

### Beispiel 3

### Darstellung von E-2-Methoxyimino-2-(2'-methylphenyl)acetonitril.

188 g (1,68 mol) trockenes Kalium-tert.-butylat werden in 2 1 trockenem Toluol vorgelegt und zügig mit 200 g (1,53 mol) ortho-Methylbenzylcyanid und 173 g (1,68 mol) tert.-Butylnitrit in 200 ml Toluol versetzt. Das Gemisch erwärmt sich auf ca. 70°C, wird 2 h nachgerührt und mit 1 1 Methyl-tert.-butylether versetzt. Das gelbe Kaliumsalz wird abgesaugt, mit Methyl-tert.- butylether nachgewaschen und bei 50°C im Vakuum getrocknet.

290 g (1,46 mol) dieses Salzes werden nun in 2,5 1 trockenem Aceton mit 20 g (0,15 mol) Kaliumcarbonat vorgelegt. Während man 234 g (1,65 mol) Jodmethan zutropft, steigt die Temperatur auf ca. 35°C. Man läßt über Nacht nachrühren und verteilt dann zwischen Wasser und Methyl-tert.-butylether. Die organische Phase wird mit Wasser gewaschen über Na₂SO₄ getrocknet, eingeengt und im Vakuum destilliert (Kp. = 95 - 100°C / 0,3 - 0,1 mm). Man erhält so 229 g (78 % Gesamtausbeute) der Titelverbindung als schwerbewegliche Flüssigkeit.
- ¹H-NMR (CDCl₃): δ =: 2,53 (s, 3H); 4,22 (s, 3H); 7,2 - 7,4 (m, 3H); 7,54 (dd, 1H) ppm.

### Beispiel 4

Darstellung von E-2-Methoxyimino-2-(2'-brommethylphenyl)acetonitril.

48 g ( 0,28 mol) E-2-Methoxyimino-2-(2'-methylphenyl)acetonitril und 54 g (0,30 mol) N-Bromsuccinimid werden in 250 ml Tetrachlorkohlenstoff vorgelegt. Es wird 40 min mit einer Hg-Dampflampe (300 W) von außen bestrahlt. Das Succinimid wird abgesaugt und die Lösung im Vakuum eingedampft. Es verbleiben 62 g (88 % Ausbeute) der Titelverbindung, die nach dem Ergebnis des ¹H-NMR-Spektrums ca. 80 %ig ist.
- ¹H-NMR (CDCl₃): δ =: 4,27 (s, 3H); 4,80 (s, 2H); 7,4 - 7,5 (m, 3H); 7,69 (dd, 1H) ppm.

### Beispiel 5

### Darstellung von 2-Methoximino-2-[(2'-brommethyl)phenyl]essigsäure-N-methylamid

10 g 2-Methoximino-2-[(2-methylphenyloxymethyl)-phenyl]essigäsure-N-methylamid werden in 50 ml Dichlormethan vorgelegt. In diese Lösung gast man bei 10°C 9 g HBr ein. Anschließend läßt man auftauen und rührt 3 Tage bei Raumtemperatur. Man gibt 50 ml Dichlormethan zu, wäscht mit 5 %iger NaOH und mit Wasser, trocknet und engt ein. Man erhält 7 g der Titelverbindung als hellbraunen Feststoff.
- m.p.:: 128 - 129°C
- ¹H-NMR (CDCl₃ / TMS):: S. 2,95 (d, 3H); 3,95 (s, 3H); 4,35 (s, 2H); 6,85 (NH); 7,1-7,5 ppm (m, 4H).

### Beispiel 6

### Darstellung von 2-Methoximino-2-[(2'-chlormethyl)phenyl]essigsäure-N-methylamid

Zu einer Lösung von 2 g (6,4 mmol) 2-Methoximino-2-[(2-methylphenyloxymethyl)-phenyl]essigsäure-N-methylamid in 30 ml Dichlormethan tropft man bei 10°C 18,8 g einer Bortrichlorid-Lösung (1 molar in n-Hexan) und erhitzt anschließend 1,5 Stunden unter Rückfluß. Anschließend wird mit 8,2 g Methanol versetzt und über Nacht bei Raumtemperatur gerührt. Man wäscht mit 5 %iger NaOH und mit Wasser, trocknet und engt ein. Es verbleiben 1,2 g (78 %) der Titelverbindung als Feststoff.
- ¹H-NMR (CDCl₃ / TMS):: δ = 2,96 (s, 3H); 3,96 (s, 3H); 4,46 (s, 2H); 6,86 (NH); 7,13-7,49 ppm (m, 4H).

### Beispiel A (Verfahren zur Herstellung der Verbindungen I)

### Darstellung von 2-Methoximino-2-[2'-1'-(4'''-methylphenyl)-1"-methyl)iminoxymethyl]phenylessigsäure-N-methylamid

Zu einer Lösung von 2 G (7 mmol) Oximetheramidbromid (aus Beispiel (5) in 20 ml Dichlormethan gibt man eine Spatelspitze Tetra-n-butylammoniumjodid und anschließend eine Lösung von 1,2 g (7 mmol) p-Methylacetophenonoxim in 10 ml Dichlormethan. Man tropft dann 20 ml 10 %ige NaOH zu und erhitzt 6 Stunden unter Rückfluß. Nach Phasentrennung wird mit Dichlormethan extrahiert, getrocknet, eingeengt, und der Rückstand mit Hexan/Methyl-tert.-butylether an Kieselgel chromatographiert. Es verbleiben 1,1 g (45 %) der Titelverbindung.
- ¹H-NMR (CDCl₃/TMS):: δ = 2,18; 2,34 (s, 3H); 2,85 (d, 3H); 3,95 (s, 3H); 5,09 (s, 2H); 6,67 (NH); 7,12-7,50 ppm (m, 8H).

### Beispiel B (Verfahren zur Herstellung der Verbindungen I) Darstellung von 4-Methoxyacetophenonoxim-O-[2-brom]benzylether

Zu einer Suspension von 2,8 g (94 mml) NaH (80 %ig) in 250 ml DMF tropft man unter Stickstoff eine Lösung von 14 g (85 mmol) p-Methocyacetophenonoxim und rührt 2 Stunden bei Raumtemperatur. Anschließend tropft man eine Lösung von 21,3 g (85 mmol) o-Brombenzylchlorid in 10 ml DMF zu und rührt 1,5 Stunden nach. Man hydrolysiert mit 10 %iger HCl und extrahiert mit Methyl-tert.-butylether. Nach Trocknen und Einengen verbleiben 27,7 g (98 %) der Titelverbindung als gelbbraunes Öl.
- ¹H-NMR (CDCl₃ / TMS):: δ = 2,21; 3,85 (s, 3H); 5,34 (s, 2H); 6,91-7,66 ppm (m, 8H).

### Beispiel C (Verfahren zur Herstellung der Verbindungen I)

### Darstellung von 2-Methoximino-2-[2'-(1'-(4'''-methoxyphenyl)1"-methyl)iminooxymethyl]phenylessigsäure-N-methylamid

Zu einer Lösung von 5 g (15 mmol) Bromid (aus Beispiel 11) in 40 ml THF gibt man bei -78°C und unter Stickstoff 6,6 g (15 mmol) n-Butyllithium ( %ige Lösung in Hexan) und läßt sofort hinterher 4,4 g (30 mmol) Oxalsäurediethylester zulaufen. Man läßt auftauen, gibt Wasser zu, extrahiert mit Methyl-tert.-butylether, trocknet und engt ein. Der Rückstand wird an Kieselgel mit Hexan/ Methyl-tert.-butylether chromatographiert. Man erhält 2,7 g (51 %) des entsprechenden α-Ketoesters als Öl.

Zu einer Lösung von 400 mg dieses Ketoesters in 10 ml THF gibt man 50 ml 40 %ige N-Methylaminlösung und erhitzt 1 Stunde auf 50°C. Anschließend gibt man 2 g Methoxyaminhydrochlorid zu und erhitzt erneut 1 Stunde auf 50°C. Man läßt abkühlen, extrahiert mit Dichlormethan, trocknet und engt ein. Es verbleiben 300 mg der Titelverbindung.
- ¹H-NMR (CDCl₃ / TMS):: δ = 2,15; 3,13; 3,77 (s, 3H); 2,86 (d, 3H); 5,06 (s, 2H); 6,84-7,56 ppm (m, 8H).

Weitere erfindungsgemäße Verbindungen der Formel I sind in der Tabelle 1 aufgeführt. Die Verbindungen sind gemäß den vorstehend beschriebenen Verfahren erhältlich.

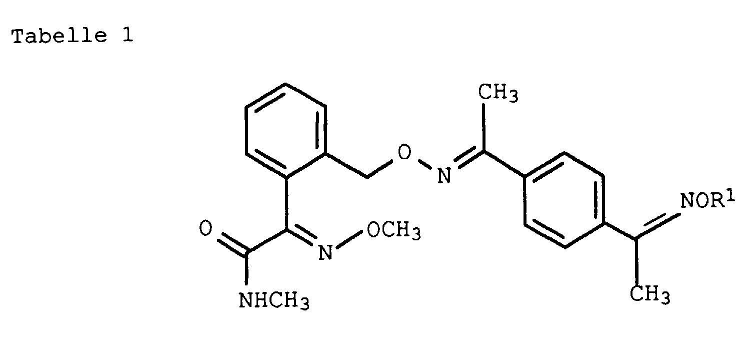

| Nr. | Aₘ | Daten |
|---|---|---|
| 1 | 4-C(CH₃)=NOCH₃ | Fp: 105 - 107°C |
| 2 | 4-C(CH₃)=NOC₂H₅ | Öl; IR (Film) : 3350, 2935, 1671, 1525, 1947, 979 |
| 3 | 4-C(CH₃)=NO-n-C₃H₇ | Öl; IR (Film) : 3350, 2963, 2935, 1672, 1525, 1037, 979, 927 |
| 4 | 4-C(CH₃)=NO-i-C₃H₇ | |
| 5 | 4-C(CH₃)=NO-n-C₄H₉ | Öl; IR (Film) : 3350, 2957, 2935, 1673, 1525, 1038, 979 |
| 6 | 4-C(CH₃)=NO-s-C₄H₉ | |
| 7 | 4-C(CH₃)=NO-i-C₄H₉ | Öl; IR (Film) : 3350, 2958, 2935, 1673, 1525, 1039, 979, 927 |
| 8 | 4-C(CH₃)=NO-t-C₄H₉ | |

Die neuen Verbindungen eignen sich als Fungizide, Insektizide.

Die erfindungsgemäßen fungiziden Verbindungen bzw. die sie enthaltenden Mittel können beispielsweise in Form von direkt versprühbaren Lösungen, Pulvern, Suspensionen, auch hochprozentigen wäßrigen, öligen oder sonstigen Suspensionen oder Dispersionen, Emulsionen, Öldispersionen, Pasten, Stäubemitteln, Streumitteln oder Granulaten durch Versprühen, Vernebeln, Verstäuben, Verstreuen oder Gießen angewendet werden. Die Anwendungformen richten sich nach den Verwendungszwecken; sie sollten in jedem Fall möglichst die feinste Verteilung der erfindungsgemäßen Wirkstoffe gewährleisten.

Normalerweise werden die Pflanzen mit den Wirkstoffen besprüht oder bestäubt oder die Samen der Pflanzen mit den Wirkstoffen behandelt.

Die Formulierungen werden in bekannter Weise hergestellt, z.B. durch Verstrecken des Wirkstoffs mit Lösungsmitteln und/oder Trägerstoffen, gewünschtenfalls unter Verwendung von Emulgiermitteln und Dispergiermitteln, wobei im Falle von Wasser als Verdünnungsmittel auch andere organische Lösungsmittel als Hilfslösungsmittel verwendet werden können. Als Hilfsstoffe kommen dafür im wesentlichen in Betracht: Lösungsmittel wie Aromaten (z.B. Xylol), chlorierte Aromaten (z.B. Chlorbenzole), Paraffine (z.B. Erdölfraktionen), Alkohole (z.B. Methanol, Butanol), Ketone (z.B. Cyclohexanon), Amine (z.B. Ethanolamin), Amide (z.B. Dimethylformamid) und Wasser; Trägerstoffe wie natürliche Gesteinsmehle (z.B. Kaoline, Tonerden, Talkum, Kreide) und synthetische Gesteinsmehle (z.B. hochdisperse Kieselsäure, Silikate); Emulgiermittel wie nichtionogene und anionische Emulgatoren (z.B. Polyoxyethylen-Fettalkohol-Ether, Alkylsulfonate und Arylsulfonate) und Dispergiermittel wie Ligninsulfitablaugen und Methylcellulose.

Als oberflächenaktive Stoffe kommen die Alkali-, Erdalkali-, Ammoniumsalze von aromatischen Sulfonsäuren, z.B. Lignin-, Phenol-, Naphthalin- und Dibutylnaphthalinsulfonsäure, sowie von Fettsäuren, Alkyl- und Alkylarylsulfonaten, Alkyl-, Laurylether-und Fettalkoholsulfaten, sowie Salze sulfatierter Hexa-, Hepta-und Octadecanolen, sowie von Fettalkoholglykolether, Kondensationsprodukte von sulfoniertem Naphthalin und seiner Derivate mit Formaldehyd, Kondensationsprodukte des Naphthalins bzw. der Naphthalinsulfonsäuren mit Phenol und Formaldehyd, Polyoxyethylenoctylphenolether, ethoxyliertes Isooctyl-, Octyl- oder Nonylphenol, Alkylphenol-, Tributylphenylpolyglykolether, Alkylarylpolyetheralkohole, Isotridecylalkohol, Fettalkoholethylenoxid-Kondensate, ethoxyliertes Rizinusöl, Polyoxyethylenalkylether oder Polyoxypropylen, Laurylalkoholpolyglykoletheracetat, Sorbitester, Lignin-Sulfitablaugen oder Methylcellulose in Betracht.

Pulver-, Streu- und Stäubemittel können durch Mischen oder gemeinsames Vermahlen der wirksamen Substanzen mit einem festen Trägerstoff hergestellt werden.

Granulate, z.B. Umhüllungs-, Imprägnierungs- und Homogengranulate können durch Bindung der Wirkstoffe an feste Trägerstoffe hergestellt werden. Feste Trägerstoffe sind Mineralerden wie Silicagel, Kieselsäuren, Kieselgele, Silikate, Talkum, Kaolin, Kalkstein, Kalk, Kreide, Bolus, Löß, Ton, Dolomit, Diatomeenerde, Calcium- und Magnesiumsulfat, Magnesiumoxid, gemahlene Kunststoffe, Düngemittel, wie Ammoniumsulfat, Ammoniumphosphat, Ammoniumnitrat, Harnstoffe und pflanzliche Produkte, wie Getreidemehl, Baumrinden-, Holz- und Nußschalenmehl, Cellulosepulver oder andere feste Trägerstoffe.

Die Verbindungen zeichnen sich durch eine hervorragende Wirksamkeit gegen ein breites Spektrum von pflanzenpathogenen Pilzen, insbesondere aus der Klasse der Ascomyceten und Basidiomyceten, aus. Sie sind zum Teil systemisch wirksam und können als Blatt-und Bodenfungizide eingesetzt werden.

Besondere Bedeutung haben sie für die Bekämpfung einer Vielzahl von Pilzen an verschiedenen Kulturpflanzen wie Weizen, Roggen, Gerste, Hafer, Reis, Mais, Gras, Baumwolle, Soja, Kaffee, Zuckerrohr, Wein, Obst- und Zierpflanzen und Gemüsepflanzen wie Gurken, Bohnen und Kürbisgewächsen, sowie an den Samen dieser Pflanzen.

Die Verbindungen werden angewendet, indem man die Pilze oder die vor Pilzbefall zu schützenden Saatgüter, Pflanzen, Materialien oder den Erdboden mit einer fungizid wirksamen Menge der Wirkstoffe behandelt.

Die Anwendung erfolgt vor oder nach der Infektion der Materialien, Pflanzen oder Samen durch die Pilze.

Speziell eignen sich die Verbindungen I zur Bekämpfung folgender Pflanzenkrankheiten:
Erysiphe graminis (echter Mehltau) in Getreide,
Erysiphe cichoracearum und Sphaerotheca fuliginea an Kürbisgewächsen,
Podosphaera leucotricha an Äpfeln,
Uncinula necator an Reben,
Puccinia-Arten an Getreide,
Rhizoctonia-Arten an Baumwolle und Rasen,
Ustilago-Arten an Getreide und Zuckerrohr,
Venturia inaequalis (Schorf) an Äpfeln,
Helminthosporium-Arten an Getreide,
Septoria nodorum an Weizen,
Botrytis cinerea (Grauschimmel) an Erdbeeren, Reben,
Cercospora arachidicola an Erdnüssen,
Pseudocercosporella herpotrichoides an Weizen, Gerste,
Pyricularia oryzae an Reis,
Phytophthora infestans an Kartoffeln und Tomaten,
Fusarium- und Verticillium-Arten an verschiedenen Pflanzen,
Plasmopara viticola an Reben,
Alternaria-Arten an Gemüse und Obst.

Die neuen Verbindungen können auch im Materialschutz (Holzschutz) eingesetzt werden, z.B. gegen Paecilomyces variotii.

Die fungiziden Mittel enthalten im allgemeinen zwischen 0,1 und 5 95, vorzugsweise zwischen 0,5 und 90 Gew.% Wirkstoff.

Die Aufwandmengen liegen je nach Art des gewünschten Effektes zwischen 0,02 und 3 kg Wirkstoff pro ha.

Bei der Saatgutbehandlung werden im allgemeinen Wirkstoffmengen von 0,001 bis 50 g, vorzugsweise 0,01 bis 10 g je Kilogramm Saatgut benötigt.

Die erfindungsgemäßen Mittel können in der Anwendungsform als Fungizide auch zusammen mit anderen Wirkstoffen vorliegen, z.B. mit Herbiziden, Insektiziden, Wachstumsregulatoren, Fungiziden oder auch mit Düngemitteln.

Beim Vermischen mit Fungiziden erhält man dabei in vielen Fällen eine Vergrößerung des fungiziden Wirkungsspektrums.

## Patentansprüche

1. N-Methylamide der Formel I, 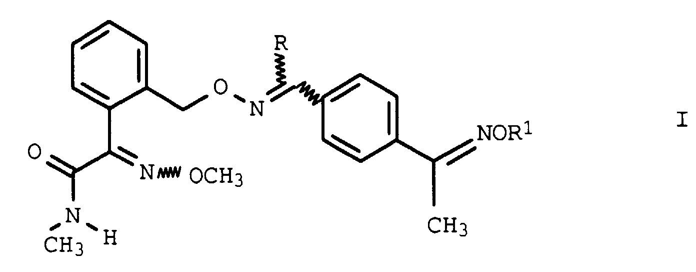 in der
R Methyl bedeutet und
R¹ eine C₁-C₄-Alkylgruppe bedeutet.

2. Verfahren zur Herstellung von N-Methylamiden der Formel I gemäß Anspruch 1, dadurch gekennzeichnet, daß man ein Benzylderivat der Formel II 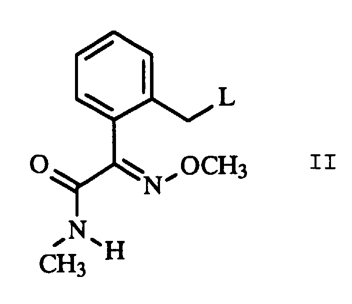 wobei L eine Abgangsgruppe ist, mit einem Oxim der Formel III

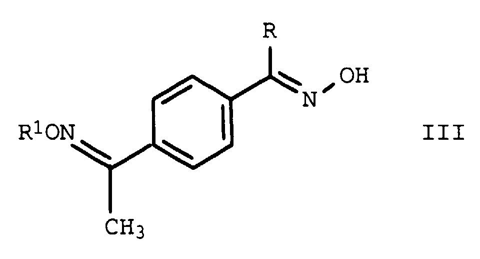

3. Verfahren zur Herstellung von N-Methylamiden der Formel I gemäß Anspruch 1, dadurch gekennzeichnet, daß man die Verbindung IV 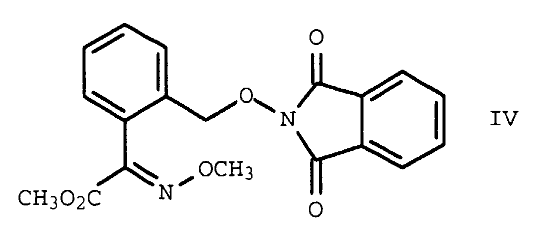 mit Methylamin in das O-Benzylhydroxylamin, V 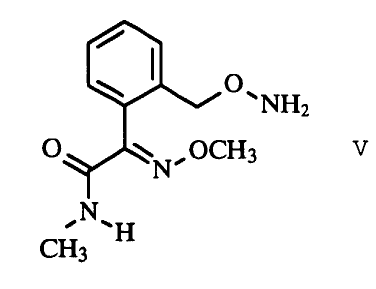 überführt und dieses mit einem Keton der Formel VI, 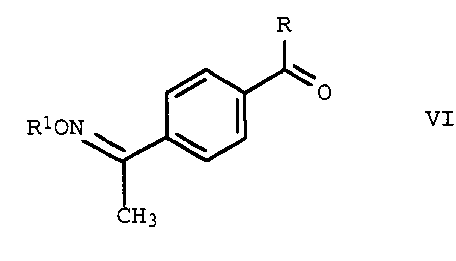 umsetzt.

4. Verfahren zur Herstellung von N-Methylamiden der Formel I gemäß Anspruch 1, dadurch gekennzeichnet, daß man Cyanoxim der Forel VII 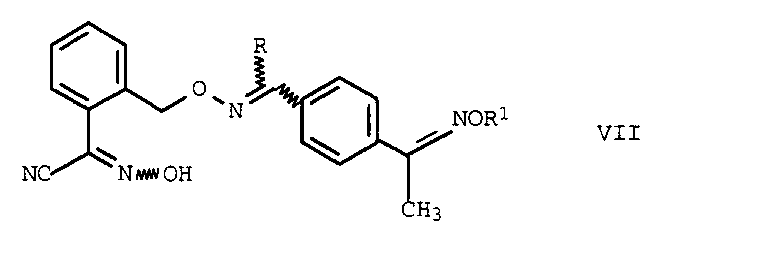 oder einen Cyanoximether der Forel VIII 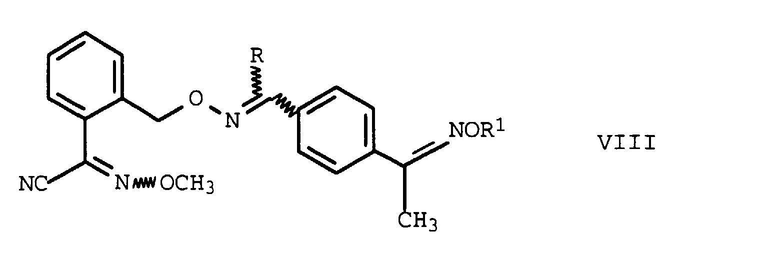 mit Methylamin umsetzt und anschließend gegebenenfalls mit einem Methylierungsmittel versetzt.

5. Verbindung der Formel V gemäß Anspruch 3.

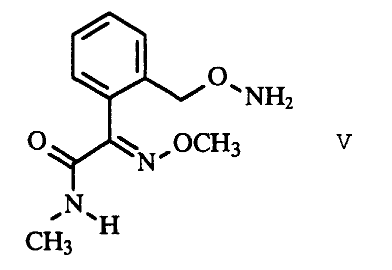

6. Verbindung der Formel IX

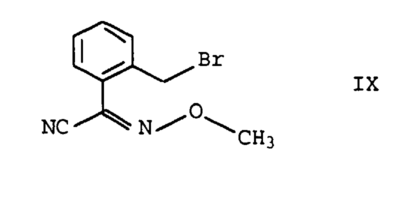

7. Fungizid, enthaltend einen inerten Trägerstoff und eine fungizid wirksame Menge eines N-Methylamids der Formel I gemäß Anspruch 1.

8. Verfahren zur Bekämpfung von Pilzen dadurch gekennzeichnet, daß man die Pilze oder die vom Pilzbefall bedrohten Materialien, Pflanzen, Saatgut oder den Erdboden behandelt mit einer fungizid wirksamen Menge einer Verbindung der Formel I gemäß Anspruch 1 behandelt.

## Claims

1. An N-methylamide of the formula I 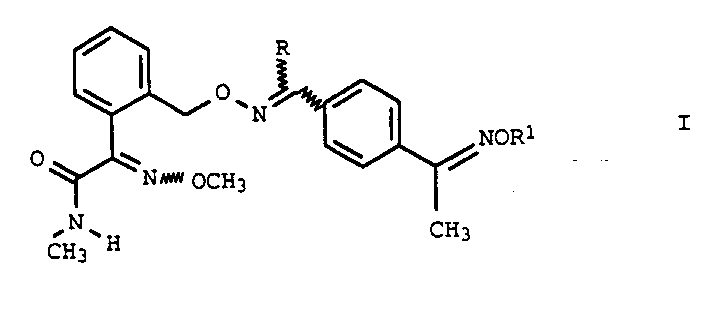 where
R is methyl and
R¹ is C₁-C₄-alkyl

2. A process for preparing N-methylamides of the formula I as claimed in claim 1, which comprises reacting a benzyl derivative of the formula II 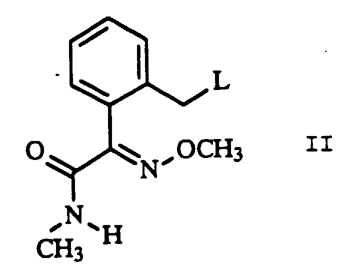 where L is a leaving group, with an oxime of the formula III

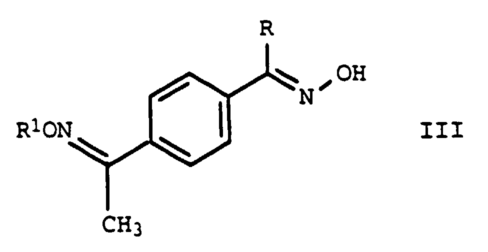

3. A process for preparing N-methylamides of the formula I as claimed in claim 1, wherein the compound IV 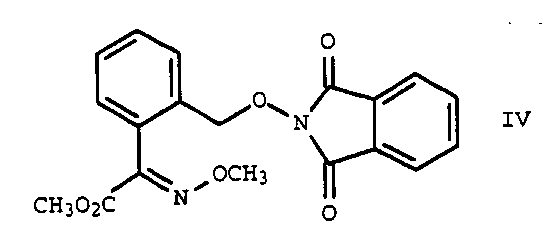 is converted using methylamine into the O-benzylhydroxylamine V 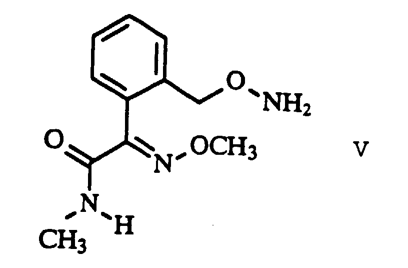 and this is reacted with a ketone of the formula VI

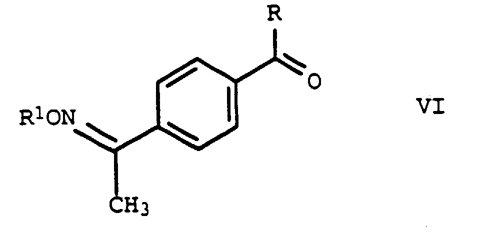

4. A process for preparing N-methylamides of the formula I as claimed in claim 1, wherein a cyanooxime of the formula VII 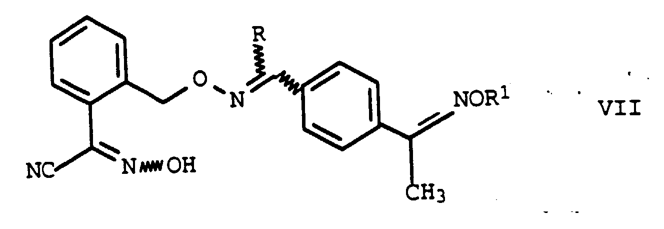 or a cyanooxime ether of the formula VIII 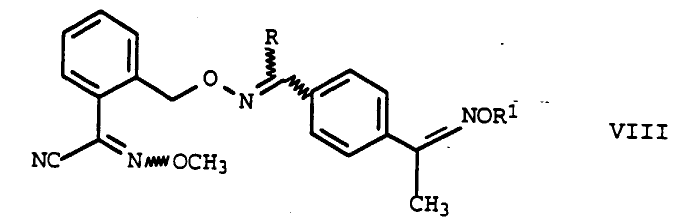 is reacted with methylamine and the product is then treated, if desired, with a methylating agent.

5. A compound of the formula V as set forth in claim 3

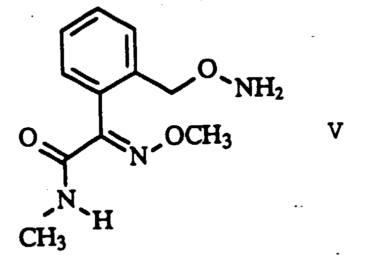

6. A compound of the formula IX

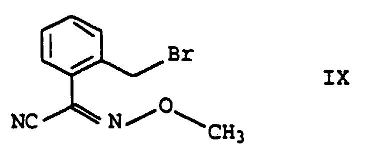

7. A fungicide containing an inert carrier and a fungicidally active amount of an N-methylamide of the formula I as claimed in claim 1.

8. A process for controlling fungi, which comprises treating the fungi or the materials, plants, seeds or the soil threatened by fungal attack with a fungicidally active amount of a compound of the formula I as claimed in claim 1.

## Revendications

1. N-méthylamides de formule I 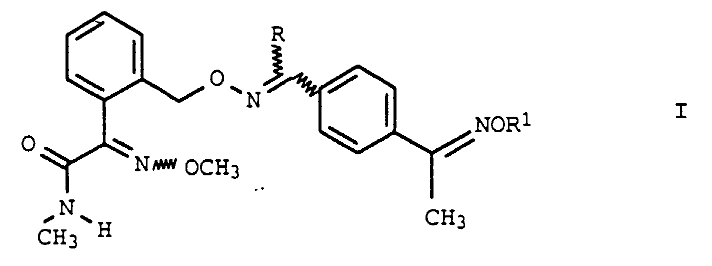 dans laquelle
R représente méthyle et
R¹ représente un groupe alkyle en C1-C4.

2. Procédé de préparation de N-méthylamides de formule I selon la revendication 1, caractérisé par le fait que l'on fait réagir un dérivé benzylique de formule II 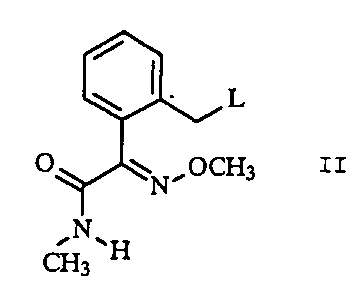 L étant un groupe éliminable, avec un oxime de formule III

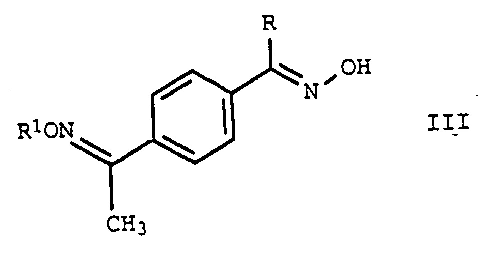

3. Procédé de préparation de N-méthylamides de formule I selon la revendication 1, caractérisé par le fait que l'on transforme le composé IV 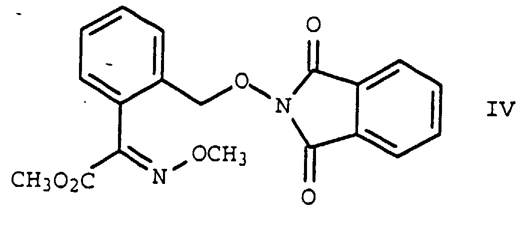 avec de la méthylamine en O-benzylhydroxylamine V 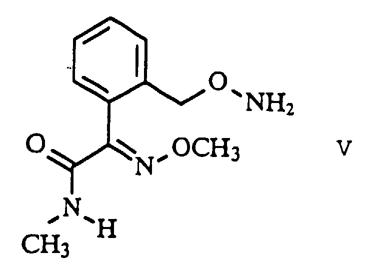 et qu'on fait réagir celle-ci avec un cétone de formule VI

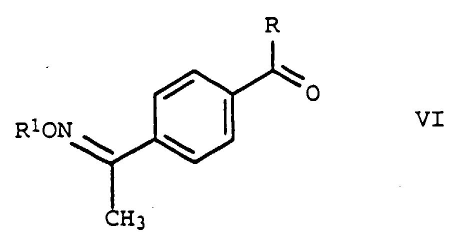

4. Procédé de préparation de N-méthylamides de formule I selon la revendication 1, caractérisé par le fait que l'on fait réagir du cyanoxime de formule VII 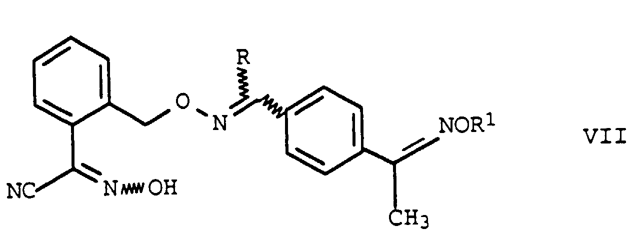 ou un cyanoximéther de formule VIII 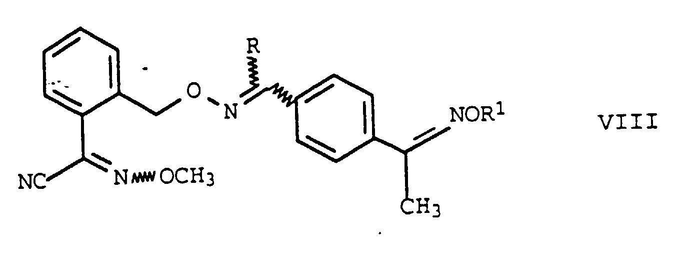 avec de la méthylamine et ensuite on additionne éventuellement avec un agent de méthylation.

5. Composé de formule V selon la revendication 3

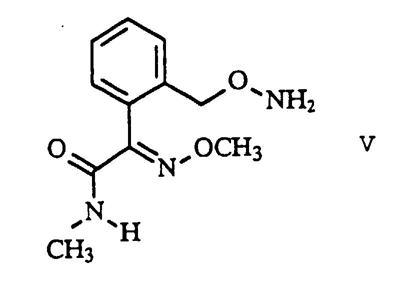

6. Composé de formule IX

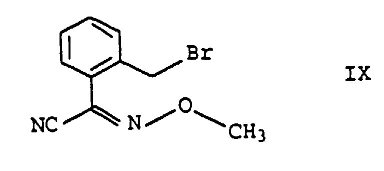

7. Fongicide contenant un support inerte et une quantité à effet fongicide d'un N-méthylamide de la formule I selon la revendication 1.

8. Procédé pour lutter contre les champignons, caractérisé par le fait que l'on traite les champignons ou les matériaux menacés d'une attaque de champignons, plantes, semences ou sols avec une quantité à effet fongicide d'un composé de formule I selon la revendication 1.
